# EUROPEAN PATENT APPLICATION

(11) **EP 2 808 679 A1**
(43) Date of publication of application: **03.12.2014**
(21) Application number: 13741512.1
(22) Date of filing: 23.01.2013
(51) Int. Cl.: G01N 33/48, G01N 1/10, G01N 1/28, G01N 33/68

(54) **DIAGNOSIS METHOD AND DIAGNOSIS SYSTEM FOR ALZHEIMER'S DISEASE**

(30) Priority: 27.01.2012 JP 2012014796
(71) Applicant: Panasonic Healthcare Co., Ltd., Ehime 791-0395 (JP); Shiga University Of Medical Science, Otsu-shi, Shiga 520-2192 (JP)
(72) Inventor: FUKUHARA, Takaomi, Osaka 540-6207 (JP); NANJO, Toshifumi, Osaka 540-6207 (JP); TOOYAMA, Ikuo, Otsu-shi Shiga 520-2192 (JP)
(74) Representative: TBK
(86) International application number: PCT/JP2013/000311
(87) International publication number: WO 2013/111578

(57) **Abstract**

Disclosed herein is a method for diagnosing Alzheimer's disease capable of minimizing invasiveness to reduce the risk of infection. The method for diagnosing Alzheimer's disease includes the steps of: pretreating an intranasal specimen; detecting tau protein or amyloid beta peptide (Aβ) in the pretreated intranasal specimen; comparing a value of the tau protein or the Aβ obtained in the detection step with a predetermined value; and displaying a comparison result obtained in the comparison step.

## Description

### Technical Field

The present invention relates to a method for diagnosing Alzheimer's disease (AD) using a specimen. More specifically, the present invention relates to a diagnosis method that achieves in-vitro diagnosis using a specimen collected from an AD patient or a healthy individual who may have AD.

### Background Art

Alzheimer's disease is a disease characterized by progressive dementia that occurs in elderly and senile age. The number of AD patients in Japan is currently said to be 1,000,000 or more, and is estimated to certainly increase in the future as the population grows older.

Clinical symptoms of Alzheimer's disease include dysmnesia, higher brain dysfunction (aphasia, apraxia, agnosia, constructive apraxia), etc. Such symptoms are often observed in other dementia diseases, and therefore it is very difficult to definitely diagnose Alzheimer's disease by clinical symptoms alone.

There has been no basic remedy for Alzheimer's disease, but since vaccine therapy in model mice succeeded in 1999, expectations for development of basic remedies have increased (see Non-Patent Document 1). In order to effectively use such basic remedies, it is necessary to diagnose Alzheimer's disease in its early stages.

On the other hand, characteristic histopathological findings of Alzheimer's disease include senile plaques and neurofibrillary tangles. The major component of the former is aggregates of amyloid beta peptide (hereinafter, referred to as Aβ) having a β-sheer structure, and the major component of the latter is excessively-phosphorylated tau protein. At present, the amyloid hypothesis that accumulation of Aβ in the brain is a significant pathological change that occurs at the onset of Alzheimer's disease is dominant (see Non-Patent Document 2). Further, it is known that histopathological changes such as accumulation of Aβ in the brain and increase in phosphorylated tau protein have started long before the onset of clinical symptoms. Therefore, detection of Aβ or tau protein in the brain as a marker is one of early diagnosis methods for Alzheimer's disease.

Based on such an idea, amyloid imaging techniques are under development, in which an image of senile plaques in the brain is taken by administering a compound, which binds to senile plaques and is modified by labeling with a radioactive substance or a substance that generates an MR signal, into the body.

Such a test is intended to directly visualize senile plaques in the brain, and therefore the degree of toxicity of the administered compound to a living body is not particularly a problem.

However, there is a case where blood sampling is performed at the same time to measure a general clinical test item, e.g., circulating thyroid hormone as a clinical indicator of cognitive dysfunction. In this case, administration of the above-described compound is undesirable because there is a possibility that a measurer is exposed to radiation.

For this reason, some methods for diagnosing Alzheimer's disease have been proposed in which the amount of Aβ accumulated in the brain is indirectly detected in blood taken from a vein and in which cerebrospinal fluid is directly collected from a patient through spinal tap to detect tau protein or the like contained in the spinal fluid (see, for example, Patent Document 1).

Patent Document 1: JP 10-509797 W

Non-Patent Document 1: Schenk D, Seubert P et al., Nature 400: 173-177, 1999
Non-Patent Document 2: Hardy JA, Selkose DF, Science 297: 353-356, 2002

### DISCLOSURE OF THE INVENTION

### Problems to be Solved by the Invention

Such conventional methods have a problem that there is a risk of infection caused by collection using a highly-invasive puncture needle. That is, the conventional methods have been expected to pose little risk because they involve collection of a material such as spinal fluid or blood and do not involve administration of a toxic substance into the body. However, after all, the conventional methods involve collection of body fluid that circulates around the body, and therefore have a high risk of infection due to the use of an invasive needle.

It is therefore an object of the present invention to provide a method for diagnosing Alzheimer's disease which is capable of minimizing invasiveness to reduce the risk of infection.

### Means for Solving the Problems

A method for diagnosing Alzheimer's disease according to the present invention includes the steps of: pretreating an intranasal specimen collected from a nasal cavity; detecting tau protein and/or amyloid beta peptide (hereinafter, referred to as Aβ) contained in the intranasal specimen pretreated in the pretreatment step; comparing a value of the tau protein and/or the Aβ obtained in the detection step with a predetermined value; and displaying a comparison result obtained in the comparison step.

### Effects of the Invention

The method for diagnosing Alzheimer's disease according to the present invention uses an intranasal specimen, which makes it possible to minimize invasiveness and to reduce the risk of infection.

### BRIEF DESCRIPTION OF THE DRAWINGS

Fig. 1(a) is a graph showing the intranasal Aβ concentrations of AD and non-AD according to Example 1, and Fig. 1(b) is a table showing the intranasal Aβ concentration values of AD and non-AD according to Example 1.
Fig. 2 (a) is a graph showing the intranasal tau protein concentrations of AD and non-AD according to Example 1, and Fig. 2(b) is a table showing the intranasal tau protein concentration values of AD and non-AD according to the Example.
Fig. 3 is a graph obtained by plotting the intranasal Aβ concentration and intranasal tau protein concentration of each subject according to Example 1.
Fig. 4 is a table showing calculated values obtained using the intranasal Aβ and tau protein concentrations of AD and non-AD according to Example 1.
Fig. 5 is a table showing the number of subjects in 6 groups classified according to the level of Alzheimer's disease based on the calculated values a obtained using the intranasal Aβ and tau protein concentrations of AD and non-AD according to Example 1 and the relationship between calculated values required for ROC.
Fig. 6 is a ROC curve diagram obtained from the calculated values obtained using the intranasal Aβ and tau protein concentrations of AD and non-AD according to Example 1.
Fig. 7 is a graph showing the intranasal Aβ concentrations of AD and non-AD according to Example 2.

### PREFERRED MODE FOR CARRYING OUT THE INVENTION

It has been previously known that Aβ is localized in nasal mucosal tissue, but it has been believed that there is no possibility that Aβ can be detected in an intranasal specimen due to its very low concentration. Further, tau protein is known as a major biomarker for AD, but has not been detected in nasal mucosal tissue by tests other than an immunohistological test using antigen-antibody reaction. In addition, there were no researchers who tried to detect tau protein in an intranasal specimen.

The present inventors have considered a novel approach for decomposing aggregates of Aβ42 and have succeeded in quantitative detection of Aβ42 and tau protein in an intranasal specimen which has been previously considered to be impossible. Therefore, the present inventors have found that the risk of infection can be reduced by minimizing invasiveness.

A method for diagnosing Alzheimer's disease according to an embodiment of the present invention includes the steps of: pretreating an intranasal specimen collected from a nasal cavity; detecting tau protein and/or amyloid beta peptide (hereinafter, referred to as Aβ) in the intranasal specimen pretreated in the pretreatment step; comparing a value of the tau protein and/or the Aβ obtained in the detection step with a predetermined value; and displaying a comparison result obtained in the comparison step.

The pretreatment step may further include the step of extracting the intranasal specimen attached to a collection tool into an extraction liquid by elution, the step of filtering the extraction liquid containing the eluted intranasal specimen for fractionation, and the step of concentrating a filtrate. In the filtration step, a filter capable of fractionation can be used. As such a filter, a filter that removes proteins of 100 kDa or more can be preferably used to obtain a filtrate containing only proteins with a molecular weight of 100 kDa or less that pass through the filter. Depending on the subsequent detection step, the concentration step may further include the step of optimizing a condition of the filtrate by, for example, neutralization for detection.

In the present invention, an extraction liquid containing formic acid is used in the extraction step to detect Aβ. The reason for this is as follows. A liquid containing body tissue (e.g., brain tissue or mucosa) where Aβ may be aggregated or adsorbed is rich in aggregated Aβ such as soluble Aβ oligomers or insoluble Aβ polymers. Therefore, when ELISA measurement is performed by directly using, for example, a measuring kit that is manufactured and sold by Wako Pure Chemical Industries, Ltd. and can quantitatively measure Aβ monomers with reliability, the amount of Aβ cannot be accurately measured.

The present inventors have tried, as a means for decomposing aggregated Aβ into Aβ monomers, a method in which formic acid is added to a solution of aggregated Aβ prepared using a standard substance.

Formic acid is generally used to solubilize proteins contained in animal or human tissue and proteins aggregated in or adsorbed to the tissue to separate them from the tissue. However, the present inventors have found that soluble Aβ oligomers are broken into Aβ monomers when formic acid acts on a liquid containing no body tissue, that is, a liquid in which soluble Aβ oligomers are already dispersed. The mechanism by which soluble Aβ oligomers are converted to Aβ monomers is unclear, but it is estimated that some soluble Aβ oligomers are probably reversibly dissociated into Aβ monomers in the liquid, and in such a state, formic acid contributes to stabilization of Aβ monomers.

In this example, formic acid is used as an additive for decomposing Aβ in the extraction step, but the additive may be a denaturant or a solubilizer, such as guanidine or urea, commonly used as a means for denaturing proteins.

The detection step will be described. The detection step involves immunoassay based on antigen-antibody reaction using, as an antigen, a substance to be measured. The immunoassay is preferably ELISA. Alternatively, the immunoassay may be performed by a method capable of directly or indirectly detecting a protein that is a substance to be measured. Preferred examples of such a method include MS (Mass Spectrometry), MS/MS, and liquid chromatography.

In the comparison step, the amount of tau protein or Aβ may be directly or indirectly calculated and then compared with a predetermined value. For example, the comparison step may be a step in which a calibration curve is created which is obtained from the standard concentration curve of target tau protein or Aβ by using values obtained by absorbance detection, fluorescence detection, luminescence detection, or electrochemical detection, the calibration curve is set as a predetermined value, an inverse regression value obtained from the calibration curve is compared with the value of tau protein or Aβ calculated based on the detection step. Here, both or one of the value of tau protein and the value of Aβ to be compared may be the logarithm of the amount of tau protein and/or the logarithm of the amount of Aβ so that both the value of tau protein and the value of Aβ are in the same range.

The displaying step involves displaying of values, diagrams, illustrations, etc. This step is preferably a step that can assist understanding by humans to judge the degree of gap between the calculated value and the predetermined value or to judge whether the calculated value is higher or lower than the predetermined value.

After the detection step, a calculation step may be performed to calculate the sum of the square of the calculated value of tau protein and the square of the calculated value of Aβ. More specifically, only the sum may be obtained as the output (result) of the calculation step, but a calculation using another parameter may be performed on the sum, or the square roots of these values or values obtained by multiplying these values by a multiplier may be obtained as the outputs of the calculation step. These outputs of the calculation step may be used as targets for comparison in the comparison step.

An embodiment in which the above-described steps are totally performed may be an integral-type diagnosis system. The diagnosis system includes: means for detecting tau protein and Aβ in an intranasal specimen collected from a nasal cavity; means for comparing values of the tau protein and the Aβ obtained by the detecting means with predetermined values, respectively; and means for displaying a comparison result obtained by the comparing means, and the above-described steps are performed on the pretreated intranasal specimen by these means in the diagnosis system to achieve diagnosis. The diagnosis system may further include means for calculating a square of a sum of a square of the value of the tau protein and a square of the value of the Aβ obtained by the detecting means, and the thus obtained calculated value is used as a value of the tau protein and the Aβ in the comparison step.

Hereinbelow, the present invention will be described in more detail with reference to examples, but the present invention is not limited to these examples.

Probable AD (hereinafter, referred to as AD) patients were diagnosed based on the clinical diagnostic criteria for Alzheimer's disease of NINCDS-ADRDA Work Group.

According to the clinical diagnostic criteria, probable AD patients are examined for dementia by three methods: clinical examination, intelligence test (e.g., Mini-Mental State Examination), and neuropsychological test, and classified into patients who have impairment in two or more cognitive functions, patients whose memory and other cognitive functions are progressively impaired, patients without impaired consciousness, patients aged 65 years or older whose age at onset is 40 to 90, and patients without systemic illness causing dementia or brain disorder other than Alzheimer's disease.

Further, findings in AD patients are progressive worsening of impairment in language, motion, and cognitive functioning (aphasia, apraxia, agnosia), impairment in activities of daily living, and change in behavior pattern. In addition, AD patients may have a family history of similar diseases (especially, pathological diagnosis of Alzheimer's disease).

Further, the diagnosis of AD in AD patents is supported by laboratory findings such as normal spinal fluid and normal brain waves or brain waves showing non-specific changes (e.g., increase in slow-waves) and by brain atrophy observed on follow-up CT (MRI).

### (Example 1)

In this example, 12 AD subjects aged 64 to 92 years with an average age of 83.2 and 9 non-AD subjects aged 29 to 65 years with an average age of 41.3 were tested. Among these subjects, 11 cases of the AD subjects aged 64 to 92 years with an average age of 83.7 and 4 cases of the non-AD subjects aged 36 to 64 years with an average age of 40.8 were measured for both Aβ and tau protein.

Hereinbelow, the measurement conditions of Aβ will be described. In this Example, nasal mucosal tissue was swabbed with a cotton swab, and a cotton ball of the cotton swab to which a nasal mucosal tissue specimen was attached was placed in a microtube to which 270 µL of ultrapure water was previously added. Then, the cotton swab was cut at a position 3.0 cm away from the tip of the cotton ball, and the ultrapure water was stirred to elute the nasal mucosal tissue specimen attached to the cotton swab.

The cotton swab is less invasive and can be used to swab a narrow and small area, and is therefore suitable for collecting a specimen from the nasal cavity. A collection tool, such as a scoop or a container, designed for collection may be used instead of a cotton swab as long as the same purpose can be achieved. Examples of such a collection tool include one that can be inserted into the nasal cavity and is equal in invasiveness to a cotton swab and one that can collect a specimen after the nasal cavity is paralyzed. Alternatively, a metering collection tool may be used which can collect a certain amount of intranasal specimen from the nasal cavity. In this example, commercially-available cotton-tipped plastic shaft swabs (cotton swabs for medical assistance HUBY-COTIX EM3-50S (manufactured by Sanyo Co., Ltd.)) were used.

The solubility of a protein greatly varies depending on the pH of a solution containing the protein, but ultrapure water does not chemically depend on pH, and therefore an extraction liquid used to elute tau protein is preferably ultrapure water. This is because a protein is generally a complex of amino acids containing a hydrophobic part and a hydrophilic part, and almost all proteins are electrically polarized and are unstable when present in solution. The ultrapure water preferably has an absorbance (210 to 400 nm) of 0.01 or less and a total organic carbon (TOC) content of 4 ppb or less.

Then, the cotton swab was raised to lift the cotton ball from the ultrapure water, and the microtube was capped to fix the cotton swab to the microtube and centrifuged at 14000 g to dewater the cotton ball. Then, the cotton swab was taken out of the microtube, and the solution in the microtube was mixed by repeatedly drawing and expelling it into and from a syringe with 27G needle 30 times. Out of the solution after mixing, 40 µL was used to measure DNA, electrolyte, and total protein. Out of the remaining solution, 200 µL was transferred to a test tube. Then, 500 µL of 100% formic acid was added to the test tube as an extraction liquid, stirred, and allowed to stand in a constant temperature bath kept at 70°C for 1 hour. After 1 hour, the test tube was taken out of the constant temperature bath, and the solution was fractionated by filtration using a filter (NANOSEP 100K OMEGA (PALL): filter that removes proteins of 100 kDa or more and allows only proteins with a molecular weight of 100 kDa or less to pass through) to obtain a filtrate. The filtrate was transferred to a test tube and concentrated under vacuum by a centrifugal evaporator until the amount thereof was reduced to 20 µL. The concentrated solution was stirred, and then 480 µL of 1 mol/L Tris (pH unadjusted) was added to prepare 500 µL of a solution. It is to be noted that each of the thus prepared solutions was measured for pH and success or failure of neutralization was recorded on recording paper, and the pH was confirmed to be in the range of about 7.0 to 8.6. These solutions were used as samples for measuring Aβ.

Aβ was measured using a measuring kit for ELISA for measuring Aβ (manufactured by Wako Pure Chemical Industries, Ltd., Human/Rat βAmyloid42 ELISA Kit wako, High-Sensitive; Item number 292-64501). Prior to measurement, solutions for creating a calibration curve were prepared using a standard solution of Aβ42 at concentrations of 0.05, 0.1, 0.2, 0.25, 1, 2, 2.5, 5, 10, and 20 pM. After the preparation, 100 µL of each of the solutions for creating a calibration curve and 1 mL of the measurement samples was added to a microtiter plate of the measuring kit.

Then, measurement was performed according to instructions for use of the measuring kit. After the measurement, the concentration of Aβ42 contained in each of the measurement samples (value of Aβ) was calculated using the calibration curve.

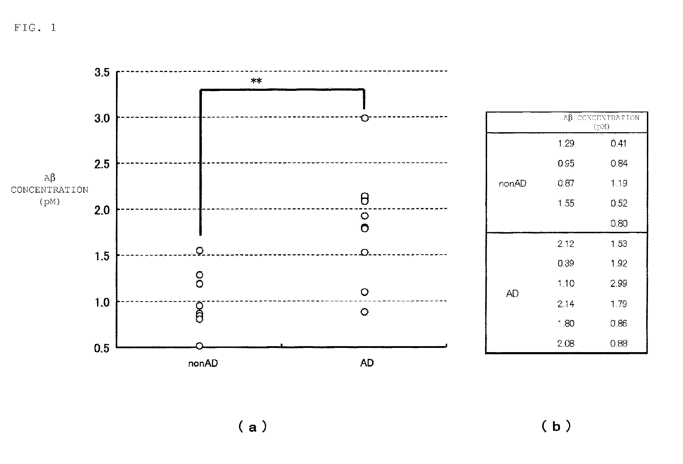

The measurement results are shown in Fig. 1. As shown in Fig. 1, each of the measurement samples had an Aβ42 value of 0.39 to 2.99 pM. In this way, the amount of Aβ contained in nasal mucosal tissue could be measured, which had never been able to be measured before. Further, there was a significant difference in the amounts of Aβs between the AD subjects and the non-AD subjects, which indicates that the amount of Aβ contained in nasal mucosal tissue is useful information for diagnosis of Alzheimer's disease.

Hereinbelow, the measurement conditions of tau protein will be described. In this example, nasal mucosal tissue was swabbed with the same cotton swab as used for Aβ measurement, and a cotton ball of the cotton swab to which a nasal mucosal tissue specimen was attached was placed in a microtube to which 150 µL of ultrapure water was previously added. Then, the cotton swab was cut at a position 3.0 cm away from the tip of the cotton ball, and the ultrapure water was stirred to elute the nasal mucosal tissue specimen attached to the cotton swab.

Then, the cotton swab was raised and fixed to the microtube in a state where the cotton ball was lifted from the ultrapure water. The microtube was set in a centrifugal machine and centrifuged at ordinary pressure for 1 minute to dewater the cotton ball, and then the cotton swab was taken out of the microtube. The thus obtained solution was used as a sample for measuring tau protein.

Tau protein was measured using a measuring kit for ELISA for measuring tau protein (manufactured by Invitrogen, Tau (Total) Human ELISA Kit; Item number: KHB0041). Prior to measurement, solutions for creating a calibration curve were prepared using a standard solution of tau protein at concentrations of 15.6, 31.2, 62.5, 125, 250, 500, 1000, and 2000 pg/mL. After the preparation, 100 µL of each of the solutions for creating a calibration curve and 1 mL of the measurement samples was added to a microtiter plate of the measuring kit.

Then, measurement was performed according to instructions for use of the measuring kit. After the measurement, the concentration of tau protein contained in each of the measurement samples (value of tau protein) was calculated using the calibration curve.

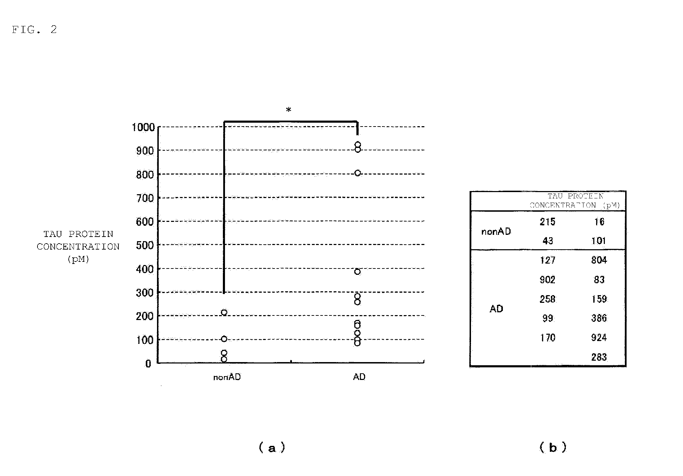

The measurement results are shown in Fig. 2. As shown in Fig. 2, each of the measurement samples had a tau protein value of 16.4 to 923.8 pM. In this way, the amount of tau protein contained in nasal mucosal tissue was first successfully detected, which had never even been considered detectable before. Further, there was a significant difference in the amounts of tau proteins between the AD subjects and the non-AD subjects, which indicates that the amount of tau protein contained in nasal mucosal tissue is useful information for diagnosis of Alzheimer's disease.

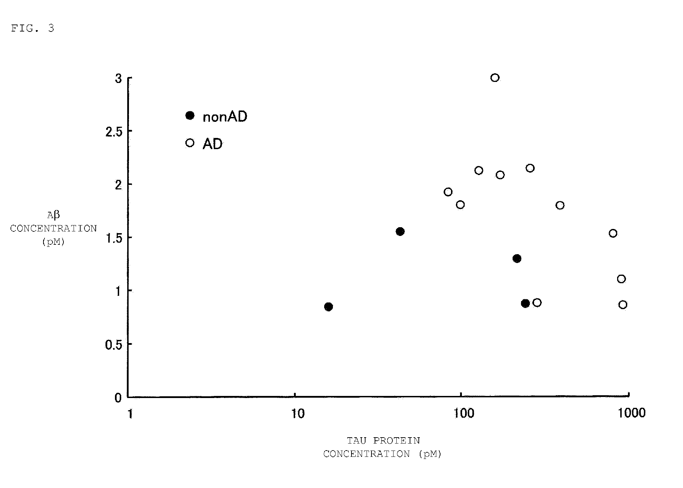

Fig. 3 is a graph obtained by plotting the data of each of the subjects obtained by the above measurement, in which the horizontal axis represents the concentration of tau protein and the axis represents the concentration of Aβ. As can be seen from the graph, the square a of the sum of the square of (Aβ concentration) and the square of (log₁₀ (tau protein concentration)), that is, the distance from the origin is a calculated value that each of the subjects can be individually obtained.

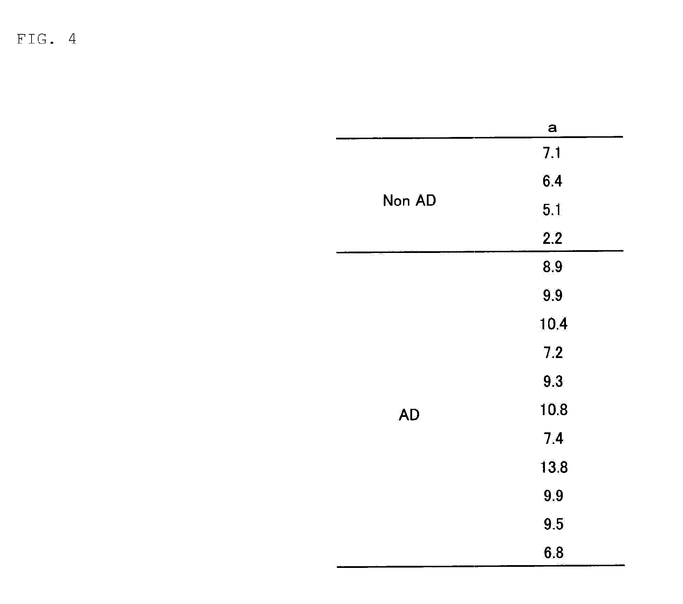

Fig. 4 shows the calculated values of the subjects who were measured for both the concentration of Aβ and the concentration of tau protein, more specifically the values of the square a, which represents the distance from the origin, of the 11 AD subjects and the 4 non-AD subjects.

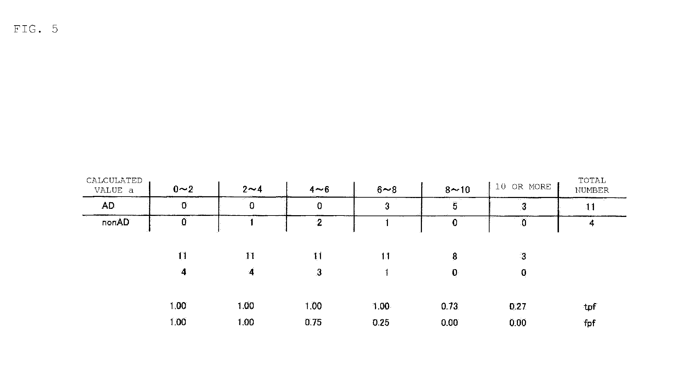

Based on the calculated values a shown in Fig. 4, the subjects were classified into 6 levels according to the level of Alzheimer's disease. The number of subjects at each level is shown in Fig. 5. More specifically, the calculated values shown in Fig. 4 were classified into 6 levels (0 to 2, 2 to 4, 4 to 6, 6 to 8, 8 to 10, and 10 or more). The number of AD subjects at each level and the number of non-AD subjects at each level are shown in the table in the upper part of Fig. 5. In the middle part of Fig. 5, the cumulative numbers of subjects are shown in order from the level "10 or more". More specifically, the number of AD subjects whose calculated value is "10 or more" is 3, the number of non-AD subjects whose calculated value is "10 or more" is 0, the number of AD subjects whose calculated value is "8 or more" is 8, the number of non-AD subjects whose calculated value is "8 or more" is 0, the number of AD subjects whose calculated value is "6 or more" is 11, the number of non-AD subjects whose calculated value is "6 or more" is 1, the number of AD subjects whose calculated value is "4 or more" is 11, the number of non-AD subjects whose calculated value is "4 or more" is 3, the number of AD subjects whose calculated value is "2 or more" or "0 or more" is 11, and the number of non-AD subjects whose calculated value is "2 or more" or "0 or more" is 4. In the lower part of Fig. 5, tpf (true positive fraction) values and fpf (false positive fraction) values are shown which were calculated from the cumulative numbers of subjects shown in the middle part of Fig. 5. The tpf value refers to a true positive rate that is a test performance indicator (value: 0 to 1) representing the ratio of the subjects correctly identified as positive by the test to all the positive (AD) subjects. In this example, when the subjects whose calculated value is "10 or more" are regarded as positive, the tpf value is 0.27 (= 3/11), when the subjects whose calculated value is "8 or more" are regarded as positive, the tpf value is 0.73 (= 8/11), and when the subjects whose calculated value is "6 or more", "4 or more", "2 or more", or "0 or more" are regarded as positive, the tpf value is 1.00. The fpf value refers to a false positive rate that is a test performance indicator (value: 0 to 1) representing the ratio of the subjects incorrectly identified as positive by the test to all the negative (non-AD) subjects. In this example, when the subjects whose calculated value is "10 or more" or "8 or more" are regarded as positive, the fpf value is 0.00, when the subjects whose calculated value is "6 or more" are regarded as positive, the fpf value is 0.25, when the subjects whose calculated value is "4 or more" are regarded as positive, the fpf value is 0.75, and when the subjects whose calculated value is "2 or more" or " 0 or more" are regarded as positive, the fpf value is 1.00.

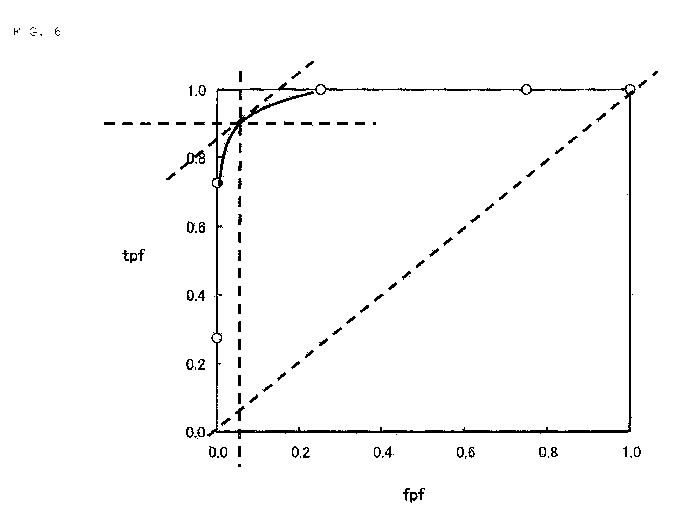

Fig. 6 is a graph showing the fpf and tpf values of the 6 cases where the subjects whose calculated value is "10 or more", "8 or more", "6 or more", "4 or more", "2 or more", and "0 or more" are regarded as positive, wherein the horizontal axis represents the fpf value and the vertical axis represents the tpf value. Further, the 6 plotted points are connected with a curve. This curve is generally called ROC curve representing specificity. It is known that when the distance from a point whose fpf value is 0 and tpf value is 1 to the ROC curve is short, that is, when the area under the ROC curve is close to 1, the accuracy of measurement is high. On the other hand, it is known that when the distance is long, that is, when the area under the ROC curve is close to 1/2 (when the ROC curve is close to a straight line shown by the dotted line in Fig. 6), the accuracy of measurement is low. As is clear from the ROC curve shown in Fig. 6, the measurement results of this example have very high accuracy.

### (Example 2)

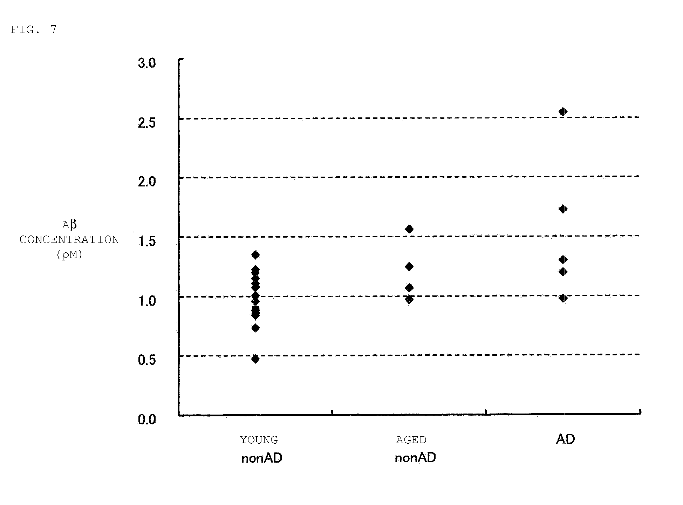

In this example, 5 AD subjects (average age: 85.6), 5 aged non-AD subjects (average age: 75.6), 15 young non-AD subjects (average age: 33) were examined in more detail, and the results will be described with reference to Fig. 7.

It is to be noted that other various measurement conditions are not repeatedly described to avoid overlaps with Example 1.

As shown in Fig. 7, the results of the young non-AD subjects are concentrated in a lower concentration range than those of the other subjects. What is important here is that there is a significant difference between the aged AD subjects and the non-AD subjects who are relatively close in age because some of the AD subjects have a relatively high Aβ concentration. That is, it is considered that measurement of the concentration of intranasal Aβ is useful to discriminate between healthy individuals and AD patients.

From these results, it has been found that discrimination between healthy people and AD patients can be achieved by detecting Aβ and tau protein in an intranasal specimen, which has been considered impossible before, and using an indicator developed based on the relationship between Aβ42 and tau protein. Therefore, information useful for diagnosis of Alzheimer's disease can be provided, which makes it possible to minimize invasiveness and to reduce the risk of infection.

Further, the use of the concentration of intranasal tau protein, newly found out by the present inventors as a result of intensive study, as an indicator of the degree of risk of development of Alzheimer's disease makes it possible to provide a diagnosis method that poses much less risk of invasion than collection of cerebrospinal fluid. Further, since the nasal cavity is close to the brain, the concentrations of Aβ and tau protein in the nasal cavity accurately and speedily reflect those in the brain, which improves the effectiveness of the diagnosis method.

### INDUSTRIAL APPLICABILITY

The present invention is useful as a method for diagnosing Alzheimer's disease. It is to be noted that in this application, diagnosis does not include professional medical practices performed by physicians but is intended to perform various steps as described above with reference to the embodiment, such as pretreatment, detection, comparison, and displaying steps using various tools, to support medical practices performed by physicians.

## Claims

1. A method for diagnosing Alzheimer's disease comprising the steps of:
pretreating an intranasal specimen collected from a nasal cavity;
detecting tau protein or amyloid beta peptide (hereinafter, referred to as Aβ) in the intranasal specimen pretreated in the pretreatment step;
comparing a value of the tau protein or the Aβ obtained in the detection step with a predetermined value; and
displaying a comparison result obtained in the comparison step.

2. The method for diagnosing Alzheimer's disease according to claim 1, wherein the pretreatment step comprises the step of extracting the intranasal specimen attached to a collection tool into an extraction liquid by elution.

3. The method for diagnosing Alzheimer's disease according to claim 2, wherein the pretreatment step comprises the step of filtering the extraction liquid containing the intranasal specimen for fractionation.

4. The method for diagnosing Alzheimer's disease according to claim 3, wherein a filter capable of fractionation is used in the filtration step.

5. The method for diagnosing Alzheimer's disease according to any one of claims 2 to 4, wherein when the tau protein in the intranasal specimen is detected, the extraction liquid is ultrapure water.

6. The method for diagnosing Alzheimer's disease according to any one of claims 2 to 4, wherein when the Aβ in the intranasal specimen is detected, the extraction liquid is formic acid.

7. The method for diagnosing Alzheimer's disease according to claim 1, wherein the intranasal specimen is collected from a nasal cavity with a cotton swab.

8. The method for diagnosing Alzheimer's disease according to claim 1, wherein the intranasal specimen is collected from a nasal cavity with a metering collection tool.

9. A method for diagnosing Alzheimer's disease comprising the steps of:
pretreating an intranasal specimen collected from a nasal cavity;
detecting tau protein and Aβ in the intranasal specimen pretreated in the pretreatment step;
comparing values of the tau protein and the Aβ obtained in the detection step with predetermined values, respectively; and
displaying a comparison result obtained in the comparison step.

10. The method for diagnosing Alzheimer's disease according to claim 9, wherein the pretreatment step comprises the step of extracting the intranasal specimen attached to a collection tool into an extraction liquid by elution, and wherein when the tau protein in the intranasal specimen is detected, the extraction liquid is ultrapure water, and when the Aβ in the intranasal specimen is detected, the extraction liquid is formic acid.

11. The method for diagnosing Alzheimer's disease according to claim 9, further comprising the step of calculating a square of a sum of squares of the values of the tau protein and the Aβ obtained in the detection step, wherein the calculated value is used as a value of the tau protein and the Aβ in the comparison step.

12. A system for diagnosing Alzheimer's disease comprising:
means for detecting tau protein and Aβ in an intranasal specimen collected from a nasal cavity;
means for comparing values of the tau protein and the Aβ obtained by the detecting means with predetermined values, respectively; and
means for displaying a comparison result obtained by the comparing means.

13. The system for diagnosing Alzheimer's disease according to claim 12, further comprising means for calculating a square of a sum of squares of the values of the tau protein and the Aβ obtained by the detecting means, wherein the obtained calculated value is used as a value of the tau protein and the Aβ in the comparison step.
